# FASCICULE DE BREVET EUROPEEN

(11) **EP 1 045 249 B1**
(45) Date de publication et mention de la délivrance du brevet: **31.12.2008**
(21) Numéro de dépôt: 00401017.9
(22) Date de dépôt: 12.04.2000
(51) Int. Cl.: G01N 33/569, B01L 3/00, C12M 3/06, G01N 1/28

(54) **Utilisation d'un dispositif de dépôt de cellules sur une plaque d'analyse**
Verwendung einer Vorrichtung zum Auftragen von Zellen auf eine Analyseplatte
Use of a device for applying cells on an analysis plate

(30) Priorité: 14.04.1999 FR 9904663
(43) Date de publication de la demande: 18.10.2000
(73) Titulaire: Peltier, Eric, 92140 Clamart (FR)
(72) Inventeur: Peltier, Eric, 92140 Clamart (FR)
(74) Mandataire: Habasque, Etienne J. Jean-François

(56) Documents cités:
- EP-A- 0 334 015
- EP-A- 0 395 430
- EP-A- 0 408 225
- AT-B- 394 455
- DE-C- 19 522 246
- GB-A- 2 291 601
- US-A- 3 783 105
- US-A- 4 693 834
- US-A- 4 895 666
- US-A- 5 081 017
- US-A- 5 593 587

## Description

La présente invention concerne l'utilisation d'un dispositif pour déposer des cellules sur une plaque d'analyse par simple décantation.

Pour le dépistage des lésions du col utérin et/ou vaginal, on réalise des prélèvements cervicaux ou vaginaux qui permettent de préparer des suspensions cytologiques en vue de leur analyse.

De façon générale, ces prélèvements peuvent être réalisés à l'aide de brosses spécifiques qui sont ensuite introduites dans un flacon contenant un fixateur cellulaire afin que les cellules prélevées soient fixées par le fixateur et forment avec celui-ci une suspension.

Les cellules doivent ensuite être déposées sur une plaque d'analyse.

On connaît déjà dans l'état de la technique, différents procédés et systèmes de dépôt pour ce type d'applications.

C'est ainsi par exemple que des moyens de centrifugation et de filtrage ont été mis en oeuvre pour obtenir ce dépôt de cellules sur la plaque. Des exemples sont décrits dans GB-A- 2 291 601 et EP-A-0408225.

Cependant, de tels moyens sont relativement complexes, encombrants, coûteux et d'une utilisation peu commode.

Le dépôt des cellules peut également être obtenu par une simple décantation. Des exemples sont décrits dans US 4 693 834 et DE-C-19522246.

Dans ce cas, la suspension cellulaire comprenant le fixateur cellulaire et les cellules, est versée dans une chambre de réception placée au-dessus de la plaque d'analyse et dont le fond est ouvert et s'étend en regard d'une zone de dépôt de cellules de la plaque d'analyse.

Les cellules se déposent alors progressivement sur la plaque, puis le fixateur est retiré de la chambre.

On conçoit cependant que cette opération de décantation est très longue.

Le but de l'invention est donc de résoudre ces problèmes.

A cet effet, on utilise un dispositif de dépôt de cellules sur une plaque d'analyse, lesdites cellules étant contenues dans une suspension cellulaire comprenant un fixateur cellulaire et lesdites cellules, ladite suspension étant versée dans une chambre de réception placée au-dessus de la plaque d'analyse, et dont le fond est ouvert et s'étend en regard d'une zone de dépôt de cellules de la plaque d'analyse, caractérisé en ce que le fond de la chambre est en communication fluidique avec un matériau d'absorption du fixateur afin d'absorber progressivement celui-ci et permettre un dépôt homogène des cellules sur la zone de dépôt de cellules de la plaque d'analyse.

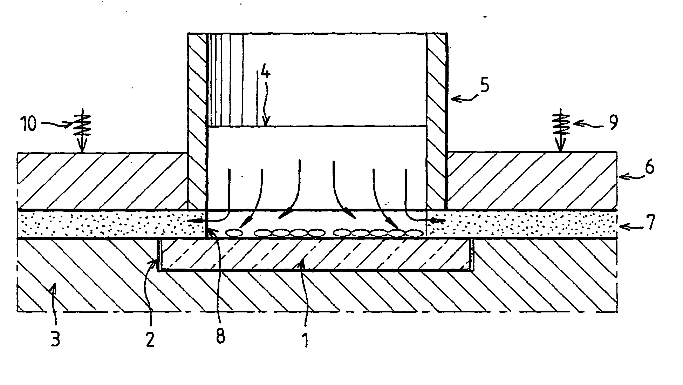

L'invention sera mieux comprise à la lecture de la description qui va suivre, donnée uniquement à titre d'exemple et faite en se référant au dessin annexé qui représente une vue en coupe schématique d'un exemple de réalisation d'un dispositif de dépôt selon l'invention.

On a en effet représenté sur cette figure, un dispositif de dépôt de cellules sur une plaque d'analyse.

La plaque d'analyse est désignée par la référence générale 1 et est formée par toute plaque appropriée déjà connue dans l'état de la technique.

Cette plaque est reçue dans une empreinte correspondante 2 d'une pièce 3 formant socle, qui sera décrite plus en détail par la suite.

Une suspension cellulaire, désignée par la référence générale 4 est versée dans une chambre de réception 5, placée au-dessus de la plaque d'analyse et dont le fond est ouvert et s'étend en regard d'une zone de dépôt de cellules de la plaque d'analyse.

Cette chambre de réception 5 est par exemple portée par une pièce de support 6 qui sera décrite plus en détail par la suite, et qui s'étend en regard de la pièce formant socle 3.

Le fond de la chambre 5 de réception de la suspension cellulaire est placé à distance de la plaque d'analyse 1 et est en communication fluidique avec un matériau d'absorption du fixateur cellulaire afin d'absorber progressivement celui-ci et permettre un dépôt homogène des cellules sur la zone de dépôt de cellules de la plaque.

Ce matériau d'absorption est désigné par la référence générale 7 sur cette figure et se présente par exemple sous la forme d'une feuille de papier buvard, munie d'un trou 8 adapté pour s'étendre en regard de la zone de dépôt de cellules de la plaque d'analyse.

Le matériau d'absorption s'étend alors autour de la zone de dépôt de cellules de la plaque entre celle-ci et la chambre de réception 5, de manière à permettre l'absorption progressive du fixateur.

Cette feuille de matériau d'absorption 7 est donc placée entre les pièces formant socle 3 et de support 6 de la chambre de réception 5 de la suspension.

Ces pièces peuvent être fixées et serrés l'une sur l'autre par l'intermédiaire de moyens de fixation et de serrage désignés par les références générales 9 et 10 sur cette figure, comprenant tout organe approprié comme par exemple des vis.

La fixation et le serrage de ces pièces l'une sur l'autre permet alors de maintenir la feuille de matériau d'absorption en position, par serrage entre celles-ci.

On conçoit également que la vitesse d'absorption du fixateur par le matériau d'absorption dépend entre autres, par exemple de la qualité de celui-ci et de la contrainte qui est exercée sur celui-ci par les pièces formant socle et de support, ce qui permet d'optimiser le dépôt de cellules de façon homogène sur la plaque d'analyse.

Il va de soi bien entendu que différents modes de réalisation de ce dispositif peuvent être envisagés, en particulier au niveau de la mise en communication fluidique du fond de la chambre de réception de la suspension cellulaire et du matériau d'absorption.

Ainsi par exemple, des entretoises délimitant un écartement prédéterminé entre la chambre et la plaque et des trajets d'absorption du fixateur, peuvent être venus de matière avec la paroi de cette chambre.

On conçoit alors que cette structure permet d'obtenir rapidement un dépôt homogène des cellules sur la plaque d'analyse, notamment sous forme de monocouche, ce qui facilite l'analyse ultérieure de celles-ci et que cette structure est particulièrement bien adaptée à l'analyse de suspensions cytologiques cervicales ou vaginales

## Revendications

1. Utilisation d'un dispositif pour déposer des cellules sur une plaque d'analyse par simple décantation, lesdites cellules étant contenues dans une suspension cellulaire (4) comprenant un fixateur cellulaire et lesdites cellules, ledit dispositif comprenant :
- la plaque d'analyse (1) ;
- un matériau (7) d'absorption du fixateur disposé autour d'une zone de dépôt de cellules de la plaque d'analyse(1) ; et
- une chambre de réception (5) prévue pour contenir la suspension, ladite chambre ayant un fond en communication fluidique avec ledit matériau d'absorption (7) afin d'absorber progressivement le fixateur et permettre un dépôt homogène des cellules sur la zone de dépôt de cellules de la plaque d'analyse (1), le fond de la chambre de réception (5) étant ouvert et s'étendant en regard de la zone de dépôt de cellules, la chambre de réception (5) étant placée au-dessus de la plaque d'analyse (1),
ledit dispositif étant **caractérisé en ce que** le fond de la chambre de réception (5) est placé à distance de la plaque d'analyse (1) pour délimiter, entre celui-ci et la plaque d'analyse (1), un trajet de communication fluidique entre cette chambre (5) et le matériau (7) d'absorption du fixateur.

2. Utilisation d'un dispositif selon la revendication 1, **caractérisé en ce que** le matériau d'absorption du fixateur se présente sous la forme d'une feuille (7) munie d'un trou (8) qui s'étend en regard de la zone de dépôt de cellules de la plaque d'analyse (1).

3. Utilisation d'un dispositif selon la revendication 1 ou 2, **caractérisé en ce qu'**il comprend deux pièces (3,6) associées à des moyens (9,10) de fixation et de serrage de l'une sur l'autre, dont l'une (6) porte la chambre de réception (5) de la suspension et l'autre (3) comporte une empreinte (2) de réception de la plaque d'analyse, et **en ce que** le matériau (7) d'absorption du fixateur est maintenu en position par serrage entre lesdites pièces (3, 6)..

4. Utilisation d'un dispositif selon l'une quelconque des revendications 1 à 3, **caractérisé en ce que** le matériau d'absorption (7) se présente sous la forme d'une feuille de papier buvard

5. Utilisation d'un dispositif selon l'une quelconque des revendications 1 à 4 pour le dépôt de cellules contenues dans une suspension cytologique cervicale.

6. Utilisation d'un dispositif selon l'une quelconque des revendications 1 à 4 pour le dépôt de cellules contenues dans une suspension cytologique vaginale.

## Claims

1. Use of a device for depositing cells on an analytical plate by simple settling, said cells being contained in a cell suspension (4) comprising a cell fixative and said cells, said device comprising:
- the analytical plate (1);
- a material (7) for absorbing the fixative, which material (7) is arranged around a cell deposition zone of the analytical plate (1); and
- a reception chamber (5) provided for containing the suspension, said chamber having a bottom which is in liquid communication with said absorption material (7) in order progressively to absorb the fixative and allow the cells to be deposited homogeneously on the cell deposition zone of the analytical plate (1), the bottom of the reception chamber (5) being open and extending opposite the cell deposition zone, the reception chamber (5) being located above the analytical plate (1),
said device being **characterised in that** the bottom of the reception chamber (5) is located at a distance from the analytical plate (1) in order to delimit, between the reception chamber (5) and the analytical plate (1), a path of liquid communication between the chamber (5) and the material (7) for absorbing the fixative.

2. Use of a device according to claim 1, **characterised in that** the material for absorbing the fixative is in the form of a sheet (7) provided with a hole (8) which extends opposite the cell deposition zone of the analytical plate (1).

3. Use of a device according to claim 1 or 2, **characterised in that** it comprises two members (3, 6) which are associated with means (9, 10) for fixing and clamping them to one another, of which one member (6) carries the reception chamber (5) for the suspension and the other (3) has a recess (2) for accommodating the analytical plate, and **in that** the material (7) for absorbing the fixative is held in position by being clamped between said members (3, 6).

4. Use of a device according to any one of claims 1 to 3, **characterised in that** the absorption material (7) is in the form of a sheet of blotting paper.

5. Use of a device according to any one of claims 1 to 4 for the deposition of cells contained in a cervical cytological suspension.

6. Use of a device according to any one of claims 1 to 4 for the deposition of cells contained in a vaginal cytological suspension.

## Patentansprüche

1. Gebrauch einer Vorrichtung zum Auftragen von Zellen auf eine Analysenplatte durch einfaches Dekantieren, wobei die genannten Zellen in einer Zellsuspension (4) enthalten sind, die einen Zellenfixierer und die genannten Zellen umfasst, wobei die genannte Vorrichtung umfasst:
- die Analysenplatte (1),
- ein Material (7) zur Absorption des Fixierers, das um eine Zellenauftragstelle der Analysenplatte (1) herum angeordnet ist, und
- eine Aufnahmekammer (5), die zur Aufnahme der Suspension vorgesehen ist, wobei die genannte Kammer einen Boden aufweist, der in Flüssigkeitsverbindung mit dem genannten Absorptionsmaterial (7) steht, um nach und nach den Fixierer zu absorbieren und ein homogenes Auftragen der Zellen an der Zellenauftragstelle der Analysenplatte (1) zu erlauben, wobei der Boden der Aufnahmekammer (5) offen ist und sich der Zellenauftragstelle gegenüber befindet, während der Boden der Aufnahmekammer (5) offen ist und sich der Zellenauftragstelle gegenüber befindet und die Aufnahmekammer (5) über der Analysenplatte (1) angeordnet ist,
wobei die genannte Vorrichtung **dadurch gekennzeichnet ist, dass** der Boden der Aufnahmekammer (5) in einem Abstand von der Analysenplatte (1) angeordnet ist, um zwischen diesem und der Analysenplatte (1) einen Flüssigkeitsverbindungsweg zwischen dieser Kammer (5) und dem Fixierer-Absorptionsmaterial (7) einzugrenzen.

2. Gebrauch einer Vorrichtung nach Patentanspruch 1, **dadurch gekennzeichnet, dass** das Fixierer-Absorptionsmaterial die Form eines Blattes (7) hat, das mit einem Loch (8) versehen ist, das sich der Zellenauftragstelle der Analysenplatte (1) gegenüber erstreckt.

3. Gebrauch einer Vorrichtung nach Patentanspruch 1 oder 2, **dadurch gekennzeichnet, dass** sie zwei Stücke (3, 6) umfasst, die mit Mitteln (9, 10) zur Befestigung und zum Anklemmen des einen ans andere verbunden sind, von denen das eine die Aufnahmekammer (5) der Suspension trägt und das andere (3) eine Eindrückung (2) zur Aufnahme der Analysenplatte, und dadurch, dass das Fixierer-Absorptionsmaterial (7) durch Einklemmen zwischen den genannten Stücken (3, 6) in Stellung gehalten wird.

4. Gebrauch einer Vorrichtung nach irgendeinem der Patentansprüche 1 bis 3, **dadurch gekennzeichnet, dass** das Absorptionsmaterial (7) die Form eines Löschpapierblattes hat.

5. Gebrauch einer Vorrichtung nach irgendeinem der Patentansprüche 1 bis 4 zum Auftragen von Zellen, die in einer Zervikalgewebe-Suspension enthalten sind.

6. Gebrauch einer Vorrichtung nach irgendeinem der Patentansprüche 1 bis 4 zum Auftragen von Zellen, die in einer Vaginalgewebe-Suspension enthalten sind.
